(19) Europäisches Patentamt
European Patent Office
Office européen des brevets

(11) EP 2 674 113 A1

(12) EUROPEAN PATENT APPLICATION

(43) Date of publication:
18.12.2013 Bulletin 2013/51

(51) Int Cl.:
*A61B 17/11* (2006.01)

(21) Application number: 13183736.1

(22) Date of filing: 29.10.2010

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 30.10.2009 IT BO20090713

(62) Document number(s) of the earlier application(s) in
accordance with Art. 76 EPC:
10793043.0 / 2 493 394

(71) Applicant: NEWMAN MEDICAL KFT.
1146 Budapest (HU)

(72) Inventor: Borghi, Enzo
40054 BUDRIO (BOLOGNA) (IT)

(74) Representative: Conti, Marco
Bugnion S.p.A.
Via di Corticella, 87
40128 Bologna (IT)

Remarks:
This application was filed on 10-09-2013 as a
divisional application to the application mentioned
under INID code 62.

(54) **Method for making an end-to-end joint for connecting end zones of body ducts**

(57) An end-to-end joint for joining end zones (2, 3) of body ducts comprises: a first joining member (4) and a second joining member (5) that can be associated with corresponding end zones (2, 3) of the duct to be joined and a connector (6) that can be associated with the two joining member (4, 5) in order to connect them to each other; each of the two joining members (4, 5) comprises a first ring (7) equipped with a plurality of rods (8) positioned along the ring's directrices; a second ring (9) coaxial with the first ring (7) and having a diameter (D9) not greater than the diameter (D7) of the first ring (7); arms (10) each having a first end (10a) connected to the first ring (7) and a second end (10b) connected to the second ring (9) to position the rings (7, 9) at a predetermined axial distance, with the rods (8) of the first ring (7) projecting towards the second ring (9); the arms (10) are plastically deformable from an open position where the rings (7, 9) are at said predetermined axial distance, to a bent position where the rings (7, 9) are close together and the rods (8) are positioned inside the second ring (9) interacting with the rods (8) to bend them in such a way that they converge towards an axis (X) of the rings (7, 9) in order to penetrate a wall of the duct (2, 3).

FIG.1

## Description

Technical Field

This invention relates to a method for making an end-to-end joint for connecting end zones of body ducts.

In other words, the invention relates to a method for making a vascular joint for anastomosis operations in which two hollow structures consisting of blood vessels or, more generally, ducts for human body fluids, are surgically connected.

Since the structures connected are two end portions of a duct for body fluids (for example, a blood vessel, a lymphatic vessel or other type of duct), the operation is called end-to-end anastomosis, precisely because the portions are connected frontally or "end-to-end" in order to restore flow to the blood vessel or duct.

Background Art

In practice, the surgeon might for example have to cut a body duct in order to perform a particular operation; at a later stage, the end portions (or free ends) of the cut duct must be connected in order to restore the continuity of the duct.

A first prior art solution for an end-to-end vascular joint (see document WO2001/72232 by the same Inventor as the present) basically comprises a first and a second joining member, both consisting of annular members configured to encompass externally the respective ends of the blood vessel or duct.

Each of these members is connected by hook-shaped elements, or staples, to the corresponding end portion of one of the blood vessels or ducts to be connected.

These two joining members are in turn joined to each other by a connector element adapted to cover the end zones of the blood vessels or ducts joined.

This joining structure, however, has a major disadvantage and that is that its metallic parts (namely, the staples) are positioned inside the blood vessel (that is to say, in contact with the fluid flowing in the duct the joint is applied to): that means there is a serious risk of creating fluid clots inside the duct which might lead to the formation of thrombi, as well as a further risk of infection.

A second prior art solution for an end-to-end vascular joint, which was intended to improve the one described above, is disclosed in patent document WO2006/043129 by the same Inventor as the present.

In the second solution, each of the two joining members is composed of two units: an annular guide member (made of a plastic material) having a plurality of through holes uniformly spaced round its circumference, and an annular staple equipped with a plurality of pointed legs.

The staple is designed to be coupled to the annular guide member by inserting the legs in the guide holes in such a way that it is substantially coaxial with the guide member. The annular guide member is made in such a way that when the legs are inserted into the guide holes, the legs are bent inwards at an angle so they are oriented according to the directrices of a cone: this allows the staple legs to penetrate the wall of the blood vessel or duct from the outside inwards but without going right through the wall of the blood vessel or duct and thus without coming into contact with the blood (or other body fluid flowing in the duct).

Obviously, in this case, too, the connection between the two joining members is secured by a connector element.

This solution also has some disadvantages, however:

- the annular member with the guide holes for the staple legs has large radial dimensions relative to the dimensions of the blood vessel or duct;
- the cost of each metal staple with the pointed legs is very high since the staple is made by a mechanical process that involves removing material from a solid block (usually cylindrical);
- the presence, for each joining member, of an element with guide holes into which must be inserted the rods associated with another part constitutes a complication for the surgeon who is applying the joint;
- the joint creates a rigid vascular connection which restrains, instead of following the pulsations of the blood vessel or the peristaltic movements of other body ducts it might be applied to.

Moreover, in the case of end-to-end vascular joints, the technical solutions adopted involve turning inside out, or everting, the edges of the end portions to be connected and then applying the joint to the everted edges of the duct.

These solutions are particularly disadvantageous because eversion of the end portions of the duct to be connected creates zones of stagnation of the fluid in the duct, leading to necrosis (with the risk of infections or other complications) of the everted portions connected, which are no longer washed by blood (where the body duct concerned is a blood vessel).

Disclosure of the Invention

This invention therefore has for an aim to overcome the above mentioned disadvantages by providing an end-to-end joint for connecting the end zones of body ducts, adapted to avoid eversion of the end zones to be connected and to prevent contact between any parts of the joint and the fluid circulating in the duct and such as to minimize the dimensions (especially the radial dimensions) of the joint itself.

Another aim of the invention is to provide an end-to-end joint for connecting the end zones of body ducts and whose application by the surgeon is made particularly easy, quick and precise.

Another aim of the invention is to provide an end-to-end joint for connecting the end zones of body ducts and which is particularly easy and inexpensive to make.

In light of this, the invention also provides a method for making the joint.

Another aim of the invention is to provide an end-to-end joint for connecting the end zones of body ducts and which follows the pulsating or peristaltic movements of the duct it is applied to.

Another aim of the invention is to provide an end-to-end joint for connecting the end zones of body ducts and which makes it easier to monitor the state of health of the person it is implanted in.

These aims are fully achieved by the joint and methods for making the joint according to the invention as characterized in the appended claims

More specifically, the end-to-end joint according to the invention, for connecting the end zones of body ducts, comprises two joining members and one connector.

According to the invention, each of the joining members comprises two rings substantially equal in diameter and joined to each other by plastically deformable arms. The first ring is equipped with pointed rods which, when the rings are pushed axially towards each other in such a way as to guide the deformation of the arms, penetrate the wall of the duct to be connected and place the joining member in a stapling condition.

The rods are guided by the second ring (inside which they are operatively inserted) in such a way as to bend towards the central axis of the second ring at an angle such that they remain within the thickness of the duct wall.

The second ring also features openings which act in conjunction with tabs on the connectors to connect the two joining members to each other. The openings also make it possible to view the duct underneath. Further, the openings facilitate penetration of the rods into the duct walls, since a predetermined pressure created inside the duct during application of the joint causes the duct wall to swell outwards at the openings.

According to another aspect of the invention, both of the rings and the connector are open, that is to say, they have a break in them, to form a sort of elastic system which makes it possible to follow the pulsations or peristaltic movements of the ducts.

According to another aspect of the invention, an end-to-end vascular joint is made from a flat strip by removing material from the flat strip (preferably by chemical etching) and then bending the machined strip into the shape of a ring to form the joining member.

Brief Description of the Drawings

The technical features of the invention according to the above mentioned aims are clearly described in the claims below and its advantages are apparent from the detailed description which follows, with reference to the accompanying drawings, which illustrate a preferred non-limiting example embodiment of the invention and in which:

- Figure 1 is an exploded perspective view of an end-to-end joint according to the invention, for connecting end zones of body ducts;
- Figure 2 is a perspective view showing the joint of Figure 1 in a compact assembled state prior to its application;
- Figure 3 is a cross section, with some parts cut away in order to better illustrate others, showing one of the joining members forming part of the joint of Figure 1 in a configuration where it is stapled to the duct;
- Figure 4 is a cross section showing the joint of Figure 1 in a configuration where it connects the two duct portions;
- Figure 5 is a plan view from above showing a portion of cut strip used to make the joining rings forming part of the joint according to the invention;
- Figure 6 is a plan view from above showing a portion of cut strip used to make a connector forming part of the joint according to the invention.

Detailed Description of the Preferred Embodiments of the Invention

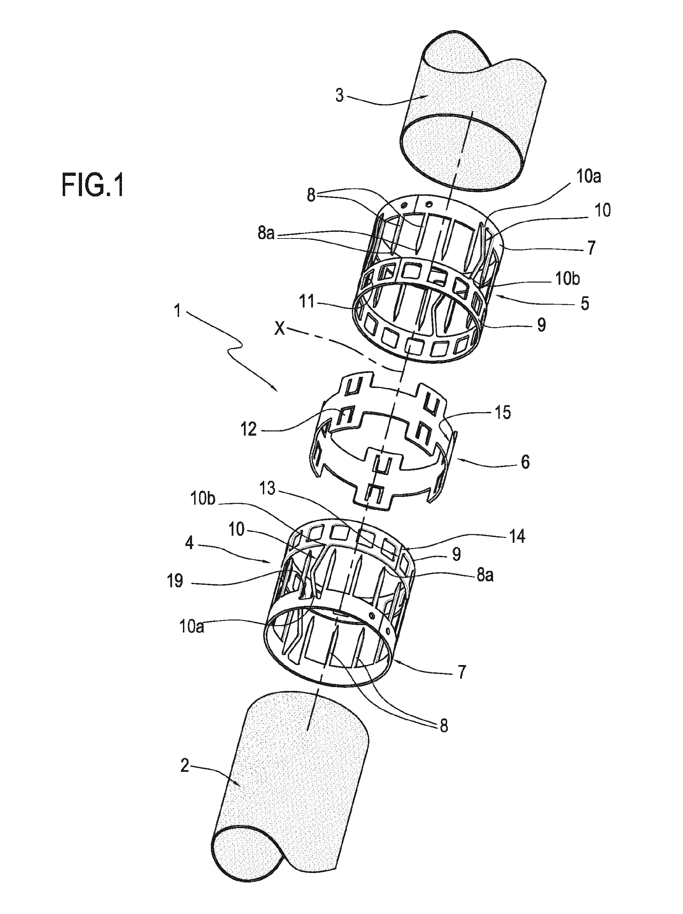

With reference to the accompanying drawings, in particular Figures 1 to 4, the end-to-end joint according to the invention, denoted in its entirety by the numeral 1, is used to connect an end zone 2 to an end zone 3 of the same body duct or, if necessary, of another duct (such as a vein, an artery or a stretch of intestine, in particular of the human body).

The joint 1 basically comprises a first and a second joining member 4, 5 that can be associated with the corresponding end zones 2, 3 of the duct to be joined, and a connector 6 that can be associated with the first and second joining members 4, 5 in order to connect them to each other when the latter are associated with the end zones 2, 3 of the duct.

As may be observed in Figures 1 to 3, each of the joining members 4 and 5 comprises: a first ring 7 and a second ring 9.

More in detail, the first ring 7 is equipped with a plurality of rods 8 positioned along directrices of the first ring 7 itself.

Preferably, the rods 8 are made as a single part with the first ring 7; also, the free ends 8a of the rods 8 are preferably tapered towards the second ring 9, that is to say, the ends are pointed so that they can penetrate a wall of the duct 2, 3 and are directed towards the second ring 9.

The second ring 9 is coaxial with the first ring 7 and has a diameter D9 which is not greater than the diameter D7 of the first ring 7, (preferably equal but possibly also slightly smaller without thereby reducing the effectiveness of the invention).

The rings 7, 9 of each joining member 4, 5 are configured to encompass externally the above mentioned duct end zones 2, 3.

According to the invention, each of the joining members 4 and 5 comprises at least two arms 10 for joining the two rings 7 and 9.

Each of the arms 10 has a first end 10a connected to the first ring 7 and a second end 10b connected to the second ring 9 to position the rings 7, 9 at a predetermined axial distance, with the rods 8 of the first ring 7 projecting towards the second ring 9.

The arms 10 are plastically deformable between an open position where the rings 7 and 9 are at said predetermined axial distance, and a bent position where the rings 7 and 9 are close together and the rods 8 are positioned inside the second ring 9 interacting with the rods 8 to bend them in such a way that they converge towards an axis X of the rings 7 and 9 in order to penetrate a wall of the duct (or rather, of the end zones 2, 3 of the duct).

It should be noted that the bent position of the arms 10 corresponds to a stapling position of the joining member 4, 5.

It should also be noted that the rings 7 and 9 are substantially cylindrical in shape, which means they define a plurality of directrices parallel to the axis X.

In the embodiment illustrated, the two rings 7 and 9 have a connecting line 13, 14 on the surface of them, along one directrix. In this embodiment, therefore, the rings 7, 9 are closed rings.

In light of this, it should be noted that the invention also contemplates an alternative embodiment (not illustrated in the drawings).

In effect, according to another aspect of the invention, the two rings 7 and 9 of each joining member 4, 5 have a break in them at the respective connecting line 13, 14 which defines two edges movable towards and away from each other to follow the pulsation of the duct 2, 3 when the joining member 4, 5 is applied to the corresponding duct 2, 3.

In light of this, it should be noted that, in the embodiment illustrated, these edges are rigidly connected (for example by laser welding).

Alternatively, the edges 13 and 14 might be connected temporarily using a bio-absorbable material which initially keeps the joining member in the position where it stably encompasses the duct 2, 3 and then, after a certain length of time, releases the edges defined by the connecting line 13 and 14 so that the rings 7 and 9 are left free to adapt to the pulsations of the fluid inside the duct.

A simpler alternative is to keep the edges free by configuring an open ring from the outset.

As regards the arms 10, there are, as illustrated, three of them and, in any case, at least three of them.

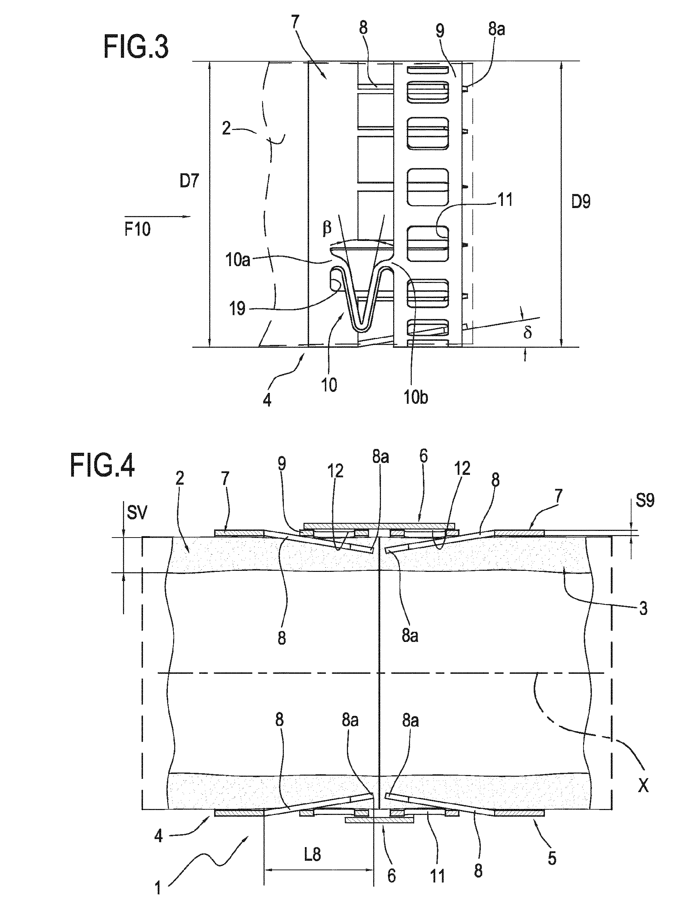

Thus, as shown in Figure 3, the arms 10 are deformable between the open position, where the rings 7 and 9 are at the predetermined axial distance, and the bent position (see Figure 3) where the rings 7 and 9 are near each other in the close-packed configuration, while the rods 8 are positioned inside the second ring 9 and are inserted in the duct wall and held within the latter without protruding to the inside of the duct where the fluid flows.

Thus, as the second ring 9 moves close to the first ring, it interacts with the rods 8 in such a way as to bend them so they converge towards the axis X and penetrate the wall of the duct 2 and 3 (see Figure 3).

In other words, therefore, the bent position of the arms 10 illustrated in Figure 3 corresponds to a stapling position of the joining members 4 and 5.

More in detail, each arm 10 comprises preferably two portions of equal length which are connected to each other uninterruptedly: that way, each arm 10 makes (that is, those portions of each arm make) an angle $\alpha$ whose amplitude varies according to the predetermined or predeterminable axial distance between the first ring 7 and the second ring 9.

Further, the two portions of each arm 10 lie in a plane tangent to the first and second rings 7 and 9 and are deformable in response to a force applied to the rings 7, 9 in axial direction (see arrow F10, Figure 3) move them close to each other into a position where the two rings 7 and 9 and the arms 10 are in a close-packed configuration.

The two portions of each arm 10 in the close-packed configuration make an angle $\beta$; this angle preferably falls within a range between 5° and 25°, and more preferably, within a range between 5° and 15°.

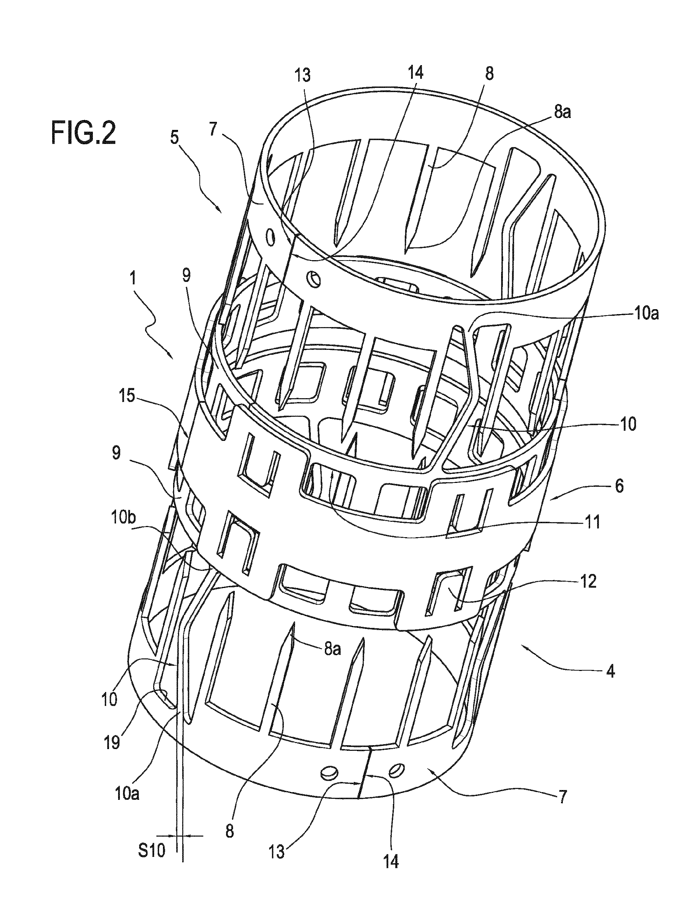

As clearly shown in Figure 2, the portion 10a of each arm 10 is connected to the first ring 7 in a portion of the first ring 7 itself, provided with a hollow 19. This creates an extension of the arm 10 portion into the hollow 19. This extension advantageously prevents the close-packed configuration of each arm 10 from reaching too small an angle (for example near 0°, that is, zero degrees) with the risk of creating on the vertex of the bent arm 10 (that is, on the central joining zone of the two arm portions) a weak zone where the arm 10 is liable to break.

Preferably, also, the first and second rings 7 and 9, the rods 8 and the arms 10 are made of the same material, for example, surgical steel.

This advantageously makes the joint particularly simple and inexpensive to produce, using the method described further on in this specification.

As stated above, when the arms 10 are in the open position, the rods 8 are within the axial dimensions defined by the two rings 7 and 9, with the pointed ends directed towards the inside edge of the second ring 9.

Preferably, the length L8 of the rods 8 is greater than the sum of the axial extension E9 of the second ring 9 plus twice the thickness S10 of the arms 10 (in a plane tangential to the outside surface of the ring, as indicated in Figure 2).

That way, the rods 8 come into contact with the second ring 9 which causes them to bend towards the axis X and to penetrate the wall of the duct 2 or 3 by a predetermined amount (see Figure 3).

Thanks to the particular configuration described, this advantageously guarantees that the pointed rods 8 penetrate well into the wall of the duct 2 or 3.

To this we must add that, to ensure that the rod 8 penetrates into the wall of the duct 2 or 3 from the outside without going through to the inside of the duct 2 or 3, the length of each rod 8 preferably satisfies a dimensional constraint expressed by the following formula (which, in practice, also determines an angle of inclination δ of the rods when in the close-packed configuration, defined by the bending of the rods 8).

$$L8 \le SV \cdot \csc\left( arctg \frac{S9}{2S10} \right)$$

In the above formula (as also visible in Figure 3):

- L8 is the length of the rod 8;
- SV is the thickness of the part of the duct 2 or 3;
- S9 is the radial thickness of the second ring 9;
- S10 is the thickness of each arm 10.

Preferably, the section size of the arms 10 is greater than that of the rods 8.

In effect, the rods 8 are configured to bend easily in the close-packed (stapling) configuration.

The thicker arms 10, on the other hand, make it possible to prevent unwanted deformation of the arms 10 themselves during application of the joining member (thus avoiding the risk of the rings 7 and 9 accidentally moving apart and causing the rods 8 to be straightened).

As illustrated in the accompanying drawings, the second ring 9 also comprises a plurality of openings 11 located along its circumference and passing through from one side to another in order to make the outside wall of the duct 2 or 3 visible when the joining member 4, 5 is applied to the respective end zone of the duct 2 or 3.

The openings 11 have a twofold function of making the end zone of the respective duct 2 or 3 visible, as just stated, and also of affording an anchoring zone for the above mentioned connector 6.

In effect, the connector 6 comprises a plurality of tabs 12 configured to interact with the openings 11 of the second ring 9 in order to form a snap fit between the connector 6 and the joining members 4 and 5.

In other terms, the tabs 12 are flexible and each of them can be fitted into a respective opening 11 on the second ring 9.

The connector 6, in the shape of a ring, presents tabs 12 alternated with empty spaces to allow a free view of a respective opening 11.

In particular, the tabs are positioned on both sides so as to be able to lock the two second rings 9 of the joining members 4 and 5.

Preferably, like each of the joining members 4 and 5, the connector 6 also has a connecting line 15 along a directrix on its surface.

As with the joining members 4 and 5 whose embodiments are described above, so the connector 6 might also be embodied in the following ways:

- the edges of the connector 6 defined by the connecting line 15 and joined (for example by laser welding), in which case the connector 6 is a closed-ring connector;
- the edges of the connector 6 defined by the connecting line 15 and joined by bio-absorbable elements, in which case the connector 6 is an open-ring or delayed/programmed opening connector.

In the second embodiment, the edges of the connector 6 are movable towards and away from each other in order to follow a pulsation of the duct 2 and 3 when the connector 6 is applied to the duct.

It should be noted that the fact that the end-to-end vascular joint comprises an annular structure with a break along a directrix to allow elastic deformation of the joint to adapt to the movements (pulsations or peristalsis) of the duct it is applied to, can be used for end-to-end vascular joints of any type.

This invention thus provides a vascular joint which originally comprises an open-ring structure.

According to another aspect of the invention, the joint 1 comprises a sensor 16 for detecting the pressure of the fluid in the duct.

The sensor 16 is associated preferably with the connector 6 of the vascular joint 1.

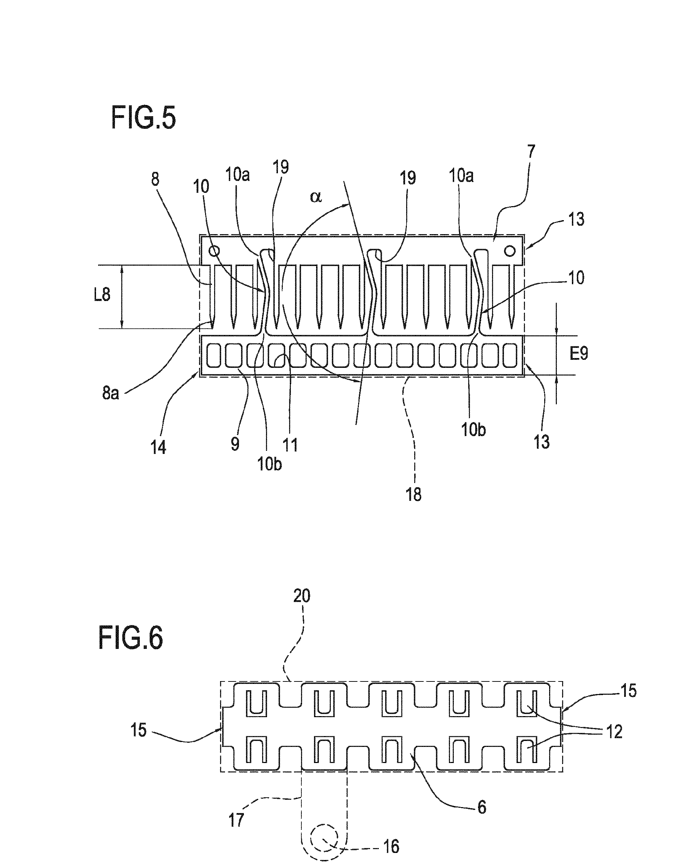

More specifically, by way of an example only, the sensor 16 for detecting the pressure may be located on a protrusion 17 on the connector 6 (drawn with a broken line in Figure 6).

It should be noted that the sensor 16 may advantageously be applied to a vascular joint of any type.

This invention thus provides a vascular joint which originally comprises a pressure sensor.

This invention also provides a method for applying the above described end-to-end joint 1 to a duct.

The method comprises the following steps:

- preparing the first and second joining members 4 and 5 and a connector 6 for joining the joining members 4 and 5;
- applying the first joining member 4 to the first end zone 2 of the duct;
- applying the second joining member 5 to the second end zone 3 of the duct;
- associating the connector 6 to the two joining members 4 and 5 in order to join them.

The step of preparing each of the joining members 4 and 5 comprises first moving the rods 8 towards the second ring 9 in such a way as to position the free ends of the rods 8 inside the second ring 9.

This advantageously makes it possible to avoid the risk that during application of the joining member 4, 5 to the duct, the rods 8 may not be positioned on the outside of the second ring 9 or may collide with it.

Application of the two joining members involves moving the two rings 7 and 9 close together by applying a force (using an applicator of substantially known type, not described since it is outside the scope of this invention) in order to plastically deform the arms 10 so that the rods 8 penetrate into the walls of the ducts 2 and 3 by interaction of the second ring 9 with the rods 8 (see Figure 3).

Lastly, application involves associating the connector 6 with the two joining members by a snap fit to engage the tabs 12 with the respective openings 11 on the second rings 9 of each joining member 4 and 5.

This invention also provides a method for making a vascular joint.

The method for making the joint 1 described above is extremely quick and practical and comprises the following steps (see Figure 5):

- preparing a flat strip 18 (whose original perimeter is shown with a dashed line);
- shaping the flat strip 18 by removing material (preferably by chemical etching or using mechanical or laser systems) to form a one-piece planar configuration of one of the joining members 4 or 5;
- deforming the shaped flat strip 18 (by bending) to obtain an annular configuration comprising the first ring and the second ring 9 and the connecting arms 10, as shown in Figure 2.

Similarly, see Figure 6, the connector 6 can also be made using the same process, that is, shaping a flat strip 20 by removing material (preferably by chemical etching) and then bending it to give the connector 6 a ring-shaped configuration.

This method has the advantage of minimizing waste, thereby reducing production costs, while at the same time allowing joints to be made very quickly.

The joint according to the invention therefore offers several advantages.

The architecture of the joining members allows the overall dimensions (especially radial dimensions) to be minimized and avoids having to evert the end portions of the duct and making the rods penetrate into the interior of the duct in contact with the fluid circulating in the duct. This is achieved by the special configuration of the joining members which allows application on the duct (with a close-packed configuration of the joining element) by plastic deformation of the arms in response to the action of moving the two rings closer together.

The possibility of having a joint with an open ring structure immediately or soon after application makes it possible for the joint to follow the pulsating or peristaltic movement of the duct even in the section where the joint is applied, thus avoiding possible narrowing of the duct passage at the joint.

The built-in pressure detecting sensor in the joint (for example applied to the connector) is designed to transmit a signal indicating the pressure of the fluid in the section where the joint is applied (this signal being detected for example in response to an elastic deformation of the joint in the open-ring embodiment of it) and allows monitoring of the state of health of the person the joint is implanted in and/or monitoring of the critical zones of the body ducts of the person (with implanted joints).

The above mentioned method of making the joint, based on planar technology to obtain the parts of the joint (for example by chemical etching) and then bending them to form the annular configuration of the joint, allows considerable savings in production costs compared to traditional technologies.

It will be understood that the invention described is susceptible of industrial application and may be modified and adapted in several ways without thereby departing from the scope of the inventive concept. Moreover, all the details of the invention may be substituted by technically equivalent elements.

**Claims**

1. A method for making a joint suitable for end-to-end joining of end zones (2, 3) of a body duct, **characterized in that** it comprises the following steps:

    - preparing a flat strip (18);
    - shaping the flat strip (18) by removing material to form a one-piece planar configuration of one of the joining members (4, 5);
    - bending the shaped flat strip (18) to obtain an annular configuration comprising a first ring (7), a second ring (9) and connecting arms (10).

2. The method according to claim 1, wherein the material is removed by chemical etching.

3. The method according to claim 1 or 2, wherein, in said annular configuration:

    - the first ring (7) is equipped with a plurality of rods (8) positioned along the ring's directrices;
    - the second ring (9) is coaxial with the first ring (7) and has a diameter (D9) not greater than the diameter (D7) of the first ring (7), said rings (7, 9) being adapted to encompass externally one of the end zones (2, 3) of the duct;
    - the arms (10) each have a first end (10a) connected to the first ring (7) and a second end (10b) connected to the second ring (9) to position the rings (7, 9) at a predetermined axial distance, with the rods (8) of the first ring (7) projecting towards the second ring (9); the arms (10) being plastically deformable.

FIG.1

FIG.2

FIG.3

FIG.4

EP 2 674 113 A1

FIG.5

FIG.6

11

Europäisches
Patentamt

European
Patent Office

Office européen
des brevets

# EUROPEAN SEARCH REPORT

Application Number

EP 13 18 3736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/269784 A1 (ABBOTT RYAN [US] ET AL) 30 October 2008 (2008-10-30) | 1,3 | INV. A61B17/11 |
| Y | * paragraphs [0144] - [0146] * | 2 | |
| Y | US 2004/097993 A1 (WHAYNE JAMES G [US]) 20 May 2004 (2004-05-20) * paragraph [0069] * | 2 | |
| A | US 2005/149075 A1 (BORGHI ENZO [IT] ET AL) 7 July 2005 (2005-07-07) * paragraph [0040] - paragraph [0049] * | 1 | |

TECHNICAL FIELDS
SEARCHED (IPC)

A61B

The present search report has been drawn up for all claims

| Place of search | Date of completion of the search | Examiner |
|---|---|---|
| Munich | 7 October 2013 | Angeli, Markus |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 13 18 3736

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2013

| Patent document cited in search report | | Publication date | Patent family member(s) | | Publication date |
|---|---|---|---|---|---|
| US 2008269784 | A1 | 30-10-2008 | NONE | | |
| US 2004097993 | A1 | 20-05-2004 | NONE | | |
| US 2005149075 | A1 | 07-07-2005 | AT | 535197 T | 15-12-2011 |
| | | | CN | 1893879 A | 10-01-2007 |
| | | | EP | 1706046 A2 | 04-10-2006 |
| | | | ES | 2374073 T3 | 13-02-2012 |
| | | | US | 2005149075 A1 | 07-07-2005 |
| | | | US | 2011066171 A1 | 17-03-2011 |
| | | | WO | 2005063130 A2 | 14-07-2005 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 200172232 A
- WO 2006043129 A